Europäisches Patentamt

(19) European Patent Office

Office européen des brevets

(11) Publication number: **0 048 989**
**B1**

(12) EUROPEAN PATENT SPECIFICATION

(45) Date of publication of patent specification: 04.09.85

(21) Application number: 81107730.4

(22) Date of filing: 29.09.81

(51) Int. Cl.⁴: **C 12 Q 1/38**, G 01 N 33/86 //
C12Q1/34

(54) Process for determining inhibitor-enzyme complexes.

(30) Priority: 30.09.80 US 192166

(43) Date of publication of application:
**07.04.82 Bulletin 82/14**

(45) Publication of the grant of the patent:
**04.09.85 Bulletin 85/36**

(84) Designated Contracting States:
**AT BE CH DE FR GB IT LI LU NL SE**

(56) References cited:
**US-A-4 180 437**

**CHEMICAL ABSTRACTS, vol. 94, no. 1, January
5, 1981, page 163, abstract 1563p Columbus,
Ohio, US P.C. HARPEL et al.
"Immunoimmobilization of alpha2-
macroglobulin-beta-trypsin complexes: a
novel approach for the biochemical
characterization of modulator-protease
interactions"**

(73) Proprietor: **CORNELL RESEARCH
FOUNDATION, INC.**
**East Hill Plaza**
**Ithaca, N.Y. 14850 (US)**

(72) Inventor: **Harpel, Peter Cahners, Dr.**
**East End Avenue 180**
**New York N.Y. 10028 (US)**

(74) Representative: **Kraus, Walter, Dr. et al
Patentanwälte Kraus Weisert & Partner Thomas-
Wimmer-Ring 15
D-8000 München 22 (DE)**

(56) References cited:
**THE JOURNAL OF IMMUNOLOGY, vol. 127, no.
1, July 1981 F.C. McDUFFIE et al. "Antigenic
changes produced by complex formation
between thrombin and antithrombin-III" pages
239-244
CHEMICAL ABSTRACTS, vol. 84, no. 11, March
15, 1976 page 168, abstract 70902d Columbus,
Ohio, US K. OHLSSON et al. "Demonstration
and semiquantitative determination of
complexes between various proteases and
human alpha2-macroglobulin"**

Courier Press, Leamington Spa, England.

CLINICAL CHEMISTRY, vol. 26, September
1980, no. 10, Easton, PA, US J. FAREED et al.
"New perspectives in coagulation testing"
pages 1380-1391

## Description

### Background of the invention

Many processes in the biological systems, e.g. animals and humans are mediated by the activation of proteinases. Some of these processes are constructive processes such as the blood clotting system; others are destructive, such as the food digesting enzymes in the gastrointestinal tract; Indirect evidence suggests that inappropriate or uncontrolled activation of proteolytic enzymes such as that which occurs in thrombosis or disseminated intravascular coagulation or other inflammatory conditions leads to tissue injury and human disease. The ability to identify and quantitate active proteinases in the blood and other body fluids has been limited by the fact that these enzymes when activated in blood or released by cells into fluids become complexed with various naturally occuring inhibitors.

At least eight distinct blood proteins have been characterized as inhibitors of protein cleaving enzymes. These inhibitors are also found in a variety of body fluids. The most important of these inhibitors appear to be alpha 2-plasmin inhibitor, alpha 2-macroglobulin, alpha 1-antitrypsin, Cl inhibitor and antithrombin-heparin cofactor. These inhibitors have a broad specificity in that each of these inhibitor proteins can form a complex with a variety of proteolytic enzymes. The ability to measure these enzyme-inhibitor complexes in fluids would aid in the diagnosis and therapy of diseases involving enzyme activation. Methods to identify and to quantitate the enzyme in complex with its inhibitor have not been satisfactory developed.

Approaches utilized by other investigators involve the production of an antibody directed against antigens in the enzyme inhibitor complex that are not shared by either one of the constituents alone. These antibodies have been produced against the alpha 2-plasmin inhibitor, plasmin complex by Plow et al. [*J. Lab. Clin. Med.,* 93: 199—209 (1979)]; and against the antithrombin, thrombin complex by several invenstigators [Lau et al., *J. Biol. Chem., 254*, 8751—8761 (1980)]. The former technique employs the detection of the plasmin-antiplasmin complex by latex agglutination assay, and the later by a radioimmunoassay. These methods are promising, however, the development of an antibody in animals directed against new antigens within the enzyme-inhibitor complex has proven to be difficult.

As pointed out hereinafter alpha 2-macroglobulin ($\alpha_2$M), proteinase complexes are a particularly useful group of complexes within the scope of this invention. The majority of proteinase complexed to $\alpha_2$M loose most of their reactivity with detecting antibodies. Thus an immunologic detecting system would not permit quantitation of the bound enzyme.

The $\alpha_2$M is a plasma protein with a wide spectrum of proteinase inhibiting activity [Harpel et al., *Progress in hermostasis and Thrombosis*, T. H. Spaet, Ed., Grune & Stratton, Inc., New York, (1976) 3: 145—189. It has been shown by a variety of investigators, as reviewed by Harpel, *supra* and Starkey et al., *Proteinases in Mammalian Cells and Tissues*, A. J. Barrett, ed., Elsevier, Amsterdam, (1977) pp. 663—696, that $\alpha_2$M binds with a remarkably varied group of protein cleaving enzymes derived from blood, circulating white blood cells, tissues, invading bacteria, plants and snake venoms. Schultz et al., Z. Naturforsch., *10b(8)*: 463—473 (1955), isolated 2M from human serum and Wallenius et al., *J. Biol. Chem., 225*: 253—267 (1957), distinguished $\alpha_2$M from IgM, the other serum globulin with a 19S sedimentation constant. Haverback et al., *J. Clin. Invest., 41*: 972—980 (1962), first demonstrated the $\alpha_2$M bound trypsin or chymotrypsin, and observed that the hydrolytic activity of the complexed enzyme was retained against low molecular weight substrates but alkost totally inhibited against large substrates such as proteins; [see also Harpel et al., *J. Clin. Invest., 52*: 2175—2184 (1973)]. The ability of $\alpha_2$M to bind proteinases without completely inhibiting the active enzymatic site of its bound enzyme is distinctive as compared to other blood proteinase inhibitors that completely inactivate the active site of the proteinases.

The identification of $\alpha_2$M-proteinase complexes in biologic fluides has been accomplished by Ohlssen et al., *Clin. Chim. Acta, 66*: 1—7 (1976), by his observation that the $\alpha_2$M enzyme complex has a different isoelectric point than does the free uncomplexed $\alpha_2$M. Other investigators, including the inventor, have isolated $\alpha_2$M from blood by chromatographic techniques and have measured the activity of the putative $\alpha_2$M enzyme complex using small molecular weight proteinase substrates.

### Brief description of the drawings

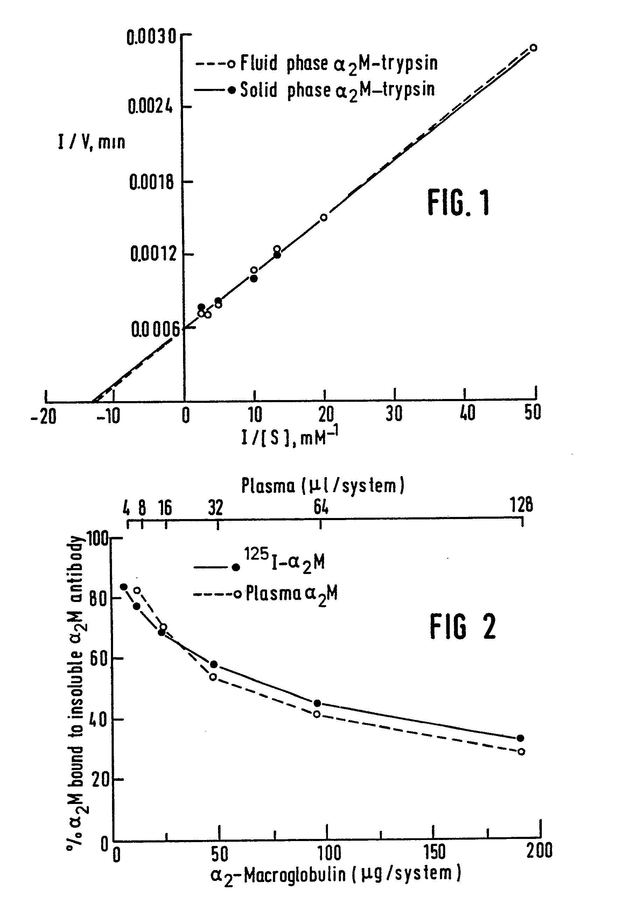

Figure 1 is a Lineweaver-Burk plot of the hydrolysis of BzPhe-Val-Arg-NHNp by fluid-phase or by solid-phase antibody-bound $\alpha_2$M-$\beta$-trypsin complexes. The hydrolytic activity of $\alpha_2$M-$\beta$-trypsin complexes was determined as indicated in the Example. All determinations are in duplicate and the points are fitted by linear regression analysis.

Figure 2 is a graphic representation of the binding of $\alpha_2$M in plasma by solid-phase $\alpha_2$M antibody. Plasma containing 1.5 mg/ml $\alpha_2$M, as determined by electroimmunoassay, was diluted with PBS. A constant quantity of $^{125}$I-$\alpha_2$M was added to each dilute (0.1 µg). The amount of plasma added per incubation system is indicated at the top of the figure. Portions (0.2 ml) were incubated in duplicate for 2 hours with solid-phase $\alpha_2$M antibody (10µl packed gel). Following separation of the gel by centrifugation the supernatant was assayed for $\alpha_2$M antigen concentration by electroimmunoassay, and $^{125}$I-$\alpha_2$M bound to the beads measured. The unlabeled $\alpha_2$M from the plasma that was bound to be immobilized antibody was determined as that

amount of $\alpha_2M$ orginally added to the antibody gel minus the remaining $\alpha_2M$ in the supernatant as measured by electroimmunoassay. The counts bound are expressed as a percent of the total counts orginally added to the immobilized antibody.

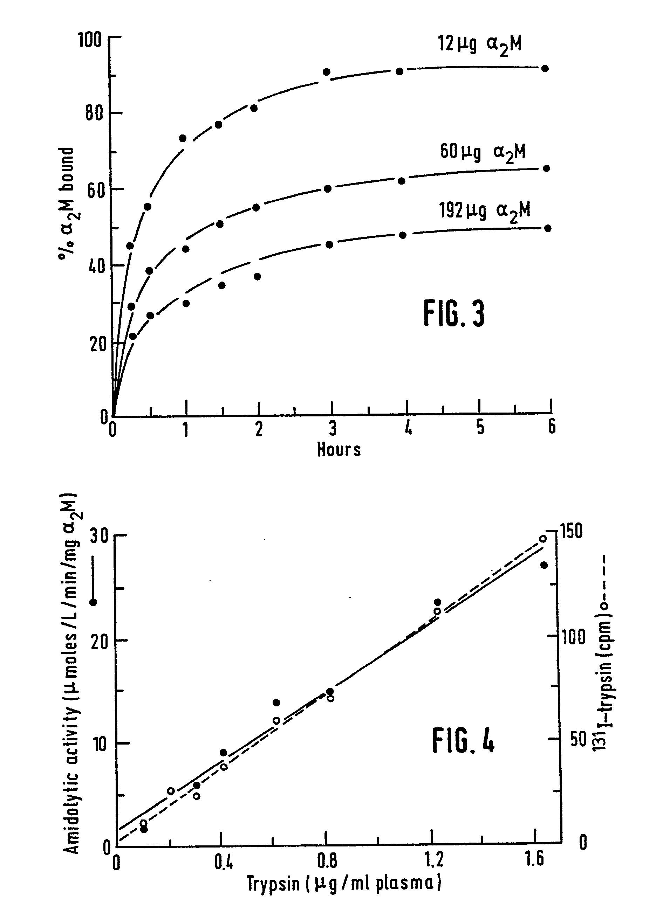

Figure 3 is a graphic representation of the effect of time in incubation on the binding of $\alpha_2M$ in plasma by solid-phase $\alpha_2M$ antibody. Plasma containing 1.5 mg/ml $\alpha_2M$ was diluted to 4, 20, and 64 % with PBS containing a trace amount of $^{125}I$-$\alpha_2M$ (0.1 µg/system). Portions (0.2 ml) were incubated in duplicate with immobilized $\alpha_2M$ antibody (10µl packed gel) and the reaction stopped by centrifugation at the intervals indicated on the abscissa. After washing four times in 1.0 ml PBS, the pellets were counted for $^{125}I$ in a gamma counter and the counts bound expressed as a percent of the total counts originally added to the insoluble antibody. The amount of $\alpha_2M$ contained in the plasma that was added to each incubation mixture is indicated.

Figure 4 is a graphic representation of the binding of plasma $\alpha_2M$-$^{131}I$-trypsin complexes by solid-phase $\alpha_2M$ antibody. Varying concentrations of $^{131}I$-trypsin (as indicated on the abscissa) were added to a mixture of $^{125}I$-$\alpha_2M$ and plasma (diluted 1/4). Following a 10 min incubation, 0.2 ml portions were mixed for 2 hours with solid-phase $\alpha_2M$ antibody (50µl packed gel). The pellets were harvested by centrifugation, washed 4 times with 1.0 ml PBS, and counted for $^{125}I$ and $^{131}I$ in a dual channel gamma counter. The gels were then assayed for amidolytic activity utilizing the substrate BzPhe-Val-Arg-NHNp as indicated in the Example. The activity is corrected for the spontaneous activity associated with the plasma to which no trypsin was added. The points are fitted by linear regression analysis.

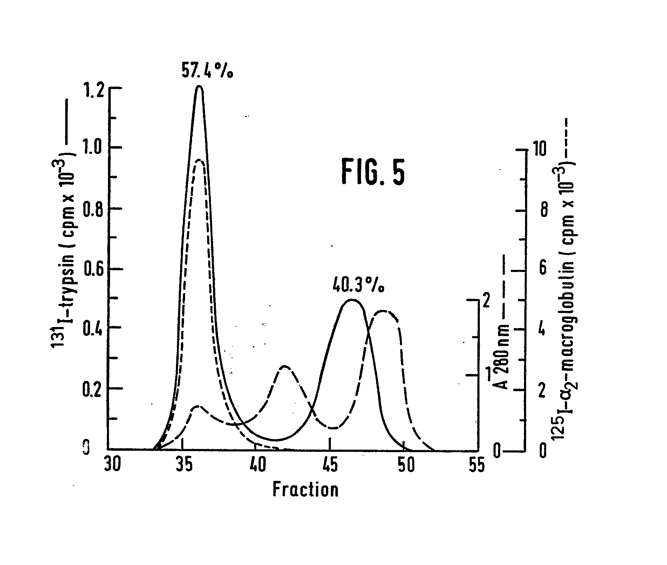

Figure 5 is a graphic representation of gel filtration chromatography of a mixture of $^{131}I$-β-trypsin $^{125}I$-$\alpha_2M$, and plasma. To 1.0 ml plasma, $^{125}I$-$\alpha_2M$ (36 µg) and $^{131}I$-β-trypsin (13 µg) were added and incubated 5 min at room temperature. The mixture was applied to a 1.4×70 cm column of Sephacryl S-200® ge filtration material (Pharmacia) maintained at 4°C in 0.05 M Tris-HCl, pH 8.0, containing 0.16 M citrate and 0.1 M NaCl. 1.6 ml fractions were collected, counted for $^{125}I$ and $^{131}I$ in a counter, and the absorbence at 280 nm measured.

Description of the invention

This invention relates to methods for detecting, identifying and quantifying enzymes, for example, proteolytic enzymes. The method broadly comprises forming an insoluble complex (E-I-anti I) comprising enzyme (E), enzyme inhibitor (I), and then detecting and identifying and preferably quantitating one or more enzymes bound to the complex. In a preferred embodiment, a matrix (M), e.g. solid or semisolid surface or permeable matrix has affixed thereto an enzyme inhibitor-antibody or enzyme inhibitor-antibody immunologically active (inhibitor- binding) fragment of such an antibody (anti I). This insoluble matrix is then contacted with biological fluid, e.g. body fluid, to bind one or more predetermined or suspected enzyme inhibitor-enzyme complexes (I-E) in the biological fluid. The bound enzyme is then detected, identified and preferably quantified.

Enzyme inhibitor (I) as defined for the purpose of this invention and useful in this invention includes not only materials which complex with the enzyme to essentially inhibit the primary functioning of the enzyme in a biological system, but also includes inhibitors such as $\alpha_2M$ which form enzyme complexes wherein the enzymatic activity of the enzyme is restricted, but not completely prevented. For example, $\alpha_2M$, proteinase complexes have been shown to retain hydrolytic activity against low molecular weight substrates, but are almost totally enzymatically nonreactive to large substrates such as proteins. Methods suitable for detecting and quantifying the bound enzyme are generally known. Generally, after the immobilized E-I-anti I complex has been removed from contact with the biological fluid the bound enzyme can be reacted in one of two general methods. The first method comprises a reaction with a detection facilitating material which reacts with a site specific to the bound enzyme or enzymes or the bound E-I complex sought and which either has a detectable group or atom measurable by an analytical technique such as radioactive tag atom, a I.R. or U.V. light absorbing group, or which causes a visible color change; or which generates a detectable cleavage product, a leaving compound or ion, which in turn is measurable by an analytical technique. The class of reaction is not dependent on the enzymatic action of the enzyme which may be biologically inactive in the E-I complex. The second method comprises an enzymatic reaction of the bound enzyme in cases such as $\alpha_2M$ where the bound enzymes retains activity against selected substrates. In such a case the immobilized E-I-anti I complex is contacted with a substrate reactive with the bound enzyme, which substrate upon reaction, by color change or the generation of a readily measurable system-unique cleavage product susceptible to analytic detection, allows analytical determination.

In some instances in the preparation of an anti I, as in the case in the Example where the IgG fraction of rabbit antihuman $\alpha_2M$ antiserum displayed activity toward an enzyme detecting substrate, it may be necessary to react, as in the example or to further purify, as through the use of chromatographic technique, the anti I to prevent interference with subsequent analysis. Alternatively, at least in some substances, this spurious activity can be measured and compensated for in the analytical determination.

It is further noted that many inhibitors are capable of binding more than one particular enzyme. Since the immobilized E-I-anti I complexes are stable, it is possible to employ *seriatim* several techniques which are capable of distinguishing between individual bound enzymes in the immobilized E-I-anti I complex. This is especially true where the detection mechanism generates a measurable leaving product rather than binding or blocking reactive sites. Again this is especially applicable to the second of the above described detecting reactions, since the substrate chosen *seriatim* may be substrates which have reactivity to only one or more specific group of enzymes potentially present in the enzymatically active E-I-anti I complex.

Given the process of the invention, quantitating the analytical results is generally within the skill of the art. At least one such method comprises quantitating I bound to the E-I-anti I complex so that final quantitation can be expressed as a function of bound inhibitor (I). This can be accomplished by adding a small amount of labeled inhibitor to the biological fluid. The amount of label in the E-I-anti I complex is then measured. This is proportional to the binding of native inhibitor.

Preferably the identification and quantification of the enzyme complexed in the immobilized inhibitor, enzyme complex is carried out in one of two ways. Where the complex is enzymatically active, as $\alpha_2M$, reaction with substrates is employed. For enzyme complexes which are not enzymatically active antibodies are prepared against the specific enzyme of interest. Thus for example, anti-bodies may be prepared in rabbits or goats against the human blood proteinases plasmin, thrombin, kallikrein, CI (the first component of complement), factor Xa, Hageman factor; or against human pancreatic enzymes trypsin, elastase or chymotrypsin; against human polymorphonuclear leukocyte (white blood cell) neutral proteinases such as elastase, collagenase and cathepsin G; or against proteinases released from damaged tissues such as cathepsin B. Such antibodies have been produced in a number of laboratories.

If desired, the immunoglobulin fraction of the antisera or the specific antibody molecules, isolated for example by affinity chromatography, are then labeled with alkaline phosphatase or other detecting enzyme by methods detailed by Engvall et al., *Immunochemistry*, 8: 871 (1971) and others, or alternatively by use of a radioactive tagging material such as $NaI^{125}$.

The insoluble immunocaptured enzyme, inhibitor complex is then incubated with a specific antienzyme antibody such as above, and after a suitable time the unbound antibody is removed by washing. The anti-enzyme antibody remains bound to the enzyme in the immobilized inhibitor complex. The anti-enzyme antibody is then measured for example by determining the activity of the alkaline phosphatase or radioactivity linked to the detecting anti-enzyme antibody. The amount of enzyme bound is quantitated, for example, using standardized curves consisting of purified inhibitor, enzyme complexes. The use of a detecting antibody *per se* is a known technique which functions well in the context of the immunocapture technique of the invention.

It is pointed out that the biological fluids being surveyed are dynamic systems in which the enzyme balance or content may change due to influences present after the state sought to be measured. For example, if the study of enzymes in a human blood sample is undertaken, it is noted the clotting grossly changes the enzymatic or E-I complex content of the blood. Thus, care must be taken in that case to prevent clotting. Likewise, to the extent possible, all enzyme or E-I complex altering factors should be avoided by the appropriate selection of reactants and equipment materials. Alternatively where possible the extraneous enzymatic changes caused during handling and processing can be accounted for and the analytical results appropriately considered or corrected.

The immobilized or insoluble complex of the invention comprises (E-I-anti I) i.e. enzyme, enzyme inhibitor and inhibitor antibody or active fragment thereof. In the preferred embodiment the complex is affixed to a solid or semi-solid matrix. The only essential requirement is that the ultimate E-I-anti I complex be immobilized. This may first result only upon the ultimate combination of E or E-I complex with the anti I to form for example a precipitate. But preferably an immobilized matrix-anti I entity is performed and then contacted with biological fluid containing E-I complex. Another possible mode is the addition of anti-I to E-I complex containing biological fluid with the subsequent immobilization, e.g. affixation to a matrix, of the preformed mobile E-I-anti I entity.

The precise nature of the matrix is not critical beyond the fact that there can be affixed thereto anti I in a manner such that the anti I moiety can be affixed thereto by some means, for example, chemical e.g. covalent bonding or absorption, adsorption, or the like, in a manner so that the anti I remains reactive with E-I complex. The matrix may be solid or permeable and may in particulate form, for example, or may comprise, for example, for at least a portion of the structure of a container or be in a unit removably associated with a container.

The biological fluid surveyed can be virtually any human, animal, microbiological, or vegetable fluid containing enzymes or E-I complexes. Body fluids such as blood, urine, or pleural, joint and peritoneal fluids can be analyzed by this technique.

Example:

This example demonstrates that purified $\alpha_2M$ as well as $\alpha_2M$-trypsin complexes are quantitatively bound to rabbit anti-human $\alpha_2M$ antibody that is immobilized on a gel matrix. This example further demonstrates that the antibody bound $\alpha_2M$-trypsin complexes possess amidolytic activity that can be readily assayed while attached to the particulate gel. The enzymatic activity of these insolubilized $\alpha_2M$-trypsin complexes is

identical to that of fluid phase $\alpha_2$M-enzyme complexes. These techniques, utilizing purified systems, have been extended to human plasma to which radiolabeled trypsin and a trace amount of radiolabeled $\alpha_2$M are added. Using the insoluble antibody technique of the invention, $\alpha_2$M-trypsin complexes are quantitatively recovered from plasma as measured both by bound radioactivity and by the capacity of the antibody-bound complex to hydrolyze a synthetic tripeptide chromogenic substrate.

Purification of $\alpha_2$-Macroglobulin

$\alpha_2$-Macroglobulin was isolated from fresh human plasma in the presence of soybean trypsin inhibitor as previously described; Harpel, *Methods Enzymol*; 45: 639—652, (1976). $\alpha_2$M was labeled with $^{125}$I by the method of McFarlane, *Biochem. J. (London)*; 62: 135—143 (1956). 0.5 mCi of carrier-free sodium $^{125}$I iodide was added to 1.7 mg $\alpha_2$M in 1.0 ml borate buffer (0.2 M) pH 8.0, containing NaCl (0.16 M). Iodine monochloride (0.05 ml containing 0.007 micromoles ICl) was added with mixing. After a 10-min incubation at room temperature, the unbound iodide was removed by Sephadex G-25® (Pharmacia) gel filtration chromatography. The specific activity of the radiolabeled $\alpha_2$M was 0.2 µCi/g.

Purification of β-trypsin

β-Trypsin was prepared from crystallized, dialyzed salt-free lyophilized bovine trypsin (Worthington) as described by Yung and Trow-bridge; *Biochem. Biophys. Res. Commun.*, 65: 927—930 (1975). The specific activity of the final product was 94 % as determined by active site titration with p-nitrophenyl-p'-guandinobenzoate HCl; Chase et al., *Methods Enzymol.*, 19: 20—27 (1970). This preparation, following reduction, consisted of a single protein band as identified by dodecyl-sulfate gel (9 %-acrylamide) electrophoresis [Weber et al., *Methods Enzymol.*, 26: 3—27 (1972)] indicating that the single chain β-form had been isolated from the original mixture of α- and β-trypsin and inactive material. The trypsin was stored at −70°C in HCl (1 mM) containing CaCl$_2$ (10 mM) and NaCl (0.1 M).

β-Trypsin was labeled with $^{131}$I by the method of McFarlane, *supra*. β-Trypsin was dialyzed against borate buffer (0.2 M), pH 8.0 containing benzamidine (0.01 M, Aldrich Chemical Co.), and NaCl (0.16 M). 0.5 mCi of carrier-free $^{131}$I-sodium iodide was added to 1.5 mg β-trypsin in a total volume of 1.0 ml of borate-benzamidine buffer. Iodine monochloride (0.05 ml containing 0.1 micromole ICl) was added with mixing. After a 10-min incubation at room temperature the free iodide and benzamidine were removed by gel filtration chromatography. The labeled pre-paration was dialyzed against HCl (1mM) con-taining NaCl (0.1 M) prior to storage at −70°C. The specific activity of the radiolabeled β-trypsin was 0.2 µCi/µg.

Preparation of rabbit antihuman $\alpha_2$M antisera

New Zealand white rabbits were immunized by intradermal injection of $\alpha_2$M mixed with Freund's adjuvant. The antisera produced one immuno-precipitation arc on double diffusion analysis against human plasma and demonstrated a reaction of identity with the starting antigen. The IgG fraction of the antisera was prepared by chromatography on DEAE-cellulose. This IgG fraction was found to hydrolyze N-benzoyl-L-phenylalanyl-L-valyl-L-arginine-p-nitro-anilide HCl (BZPhe-Val-Arg-NHNp; S-2160), however the activity was inhibited by treatment of the IgG material with α-N-p-tosyl-L-lysine chloromethyl ketone HCl (TLCK) (0.01 M) for 3 days at 4°C followed by extensive dialysis. The TLCK treated IgG was coupled to Bio-Gel A-5m® (Bio-Rad Laboratories) by the cyanogen-bromide method as detailed by March et al., *Anal. Biochem.*, 60: 149—152 (1974). Coupling to the activated gel was carried out in citrate buffer (0.2 M), pH 6.5, using 6.0 mg IgG per ml activated gel. Coupling efficiency was greater than 90 %. After the coupling procedure, the gel was incubated 1 h in 1.0 M ethanolamine pH 8.0 to neutralize any remaining protein binding groups. The IgG fractions of normal rabbit serum, and rabbit antisera directed against human alumin or human haptoglobin were also prepared and coupled to Bio-Gel A-5m as detailed above.

Studies of the binding of $\alpha_2$M to immobilized rabbit anti-human $\alpha_2$M antibody

The gel containing the bound $\alpha_2$M antibody was diluted twofold (V/V) with phosphate buffer (0.05 M), pH 7.2, containing NaCl (0.1 M) (PBS). This coupled antibody was incubated with $^{125}$I-$\alpha_2$M, $^{125}$I-$\alpha_2$M-β-trypsin complexes, or with plasma containing a trace quantity of $^{125}$I-$\alpha_2$M and varying concentrations of $^{131}$I-β-trypsin as indicated in the figure legends. All incubations, were at room temperature, in volumes of 1.0 ml or less, with constant mixing by end over end inversion using a Labquake® mixer (Labindustries, Berkeley, CA). The incubation was terminated by centrifugation and the pelletted insoluble $\alpha_2$M antibody gel washed repeatedly with 1.0 ml portions of PBS. The pellets were counted for associated $^{125}$I or $^{131}$I radioactivity in a Searle 1185 dual channel counter.

Assay of the amidolytic activity of $\alpha_2$M-β-trypsin complexes in the fluid phase or bound to immobilized $\alpha_2$M antibody

Soluble trypsin or $\alpha_2$M-trypsin complexes were assayed by methods similar to those previously detailed; Svendsen et al, *Thromb. Res.*, 1: 267—278 (1972). The substrate N-benzoyl-L-phenylalanyl-L-valyl-L-arginine-p-nitroanilide hydrochloride (BzPhe-Val-Arg-NHNp=S-2160, obtained from Ortho Diagnostics, Inc., or from Vega Biochemicals) was dissolved in distilled water (0.7 mg/ml). β-Trypsin, or $\alpha_2$M-trypsin complexes were made to a volume of 0.4 ml with Tris-HCl (0.1 M), pH 8.3, containing CaCl$_2$ (1.25 mM). Substrate (0.2 ml) was added and the mixtures were incubated at room temperature.

The reaction was terminated at varying intervals by the addition of 30 % acetic acid (0.2 ml) followed by the addition of 0.6 ml of the Tris-CaCl$_2$ buffer to achieve a final volume of 1.4 ml. The absorbance at 405 nm was measured with a Gilford 240 spectrophotometer. The concentration of p-nitroanalide released was determined using a molar absorbancy of 10,500; Aurell et al., *Thromb. Res., 11:* 595—609 (1977).

To determine the amidolytic activity of $\alpha_2$M-trypsin complexes bound to gel-coupled rabbit anti-human $\alpha_2$-trypsin complexes bound to gel-coupled rabbit anti-human $\alpha_2$M IgG, Tris-CaCl$_2$ buffer (0.4 ml) and BzPhe-Val-Arg-NHNp (0.2 ml) were added to the immobilized antibody gels that had been incubated with $\alpha_2$M-trypsin complexes. After incubation for varying time periods at room temperature, the antibody containing gel was removed by centrifugation. 30 % acetic acid (0.2 ml) was added to the supernatant, followed by the addition of 0.6 ml Tris-CaCl$_2$ buffer. The absorbance at 405 nm was measured, and the results expressed (following correction for the substrate bland) either as $\mu$ moles p-nitroanilide released/liter/min, or when the amount of $\alpha_2$M bound was determined, as $\mu$ moles/liter/min/mg $\alpha_2$M.

For the derivation of Michaelis-Menten constants, $\beta$-trypsin (0.25 $\mu$g/ml), $\alpha_2$M (50 $\mu$g/ml), and a trace quantity of $^{125}$I-$\alpha_2$M were incubated at 0°C in PBS. Assay of this incubation mixture for free $\beta$-trypsin, using the high molecular weight particulate substrate Remazol-brilliant blue hide [Hayes et al., *The Physiological Inhibitors of Blood Coagulation and Fibrinolysis,* Collen et al., eds., Elsevier, Amsterdam, 1979, pp. 273—280], proved negative and indicated that all of the $\beta$-trypsin had been bound by the $\alpha_2$M. Portions (0.2 ml) of the $\alpha_2$M-$\beta$-trypsin incubation mixture were then incubated in duplicate with rabbit anti $\alpha_2$M-IgG coupled to Bio-Gel A-5m® (10 $\mu$l packed gel). The duplicate gel incubation mixtures were washed four times each with 1.0 ml PBS and the $^{125}$I, a measure of $\alpha_2$M bound to the gel, was determined. The insolubilized $\alpha_2$M-$\beta$-trypsin complexes were then assayed in duplicate for the ability to hydrolyze varying concentrations of BzPhe-Val-Arg-NHNp. Due to the fact that continuous kinetic measurements could not be made with the insolubilized $\alpha_2$M-enzyme complexes, duplicate assays were terminated at discrete time intervals of 5, 10, and 15 minutes at each substrate concentration and the concentration of liberated p-nitroanilide determined.

Portions of the original soluble $\alpha_2$M-$\beta$-trypsin mixtures, containing an amount of radioactivity equivalent to that bound to the antibody gels described above were assayed in duplicate at varying substrate concentrations. In this case, production of p-nitroanalide was followed continuously at 405 nm in a Gilford 240 recording spectrophotometer equipped with a Honeywell 1800 recorder. Km and Vmax for the soluble and immobilized $\alpha_2$M-$\beta$-trypsin complexes were obtained from Lineweaver-Burk linear transformations of the initial reaction velocities and substrate concentrations.

Electroimmunoassay

The concentration of native $\alpha_2$M in plasma, or in plasma supernatants following incubation with immobilized $\alpha_2$M antibody was determined by the electroimmunoassay, rocket technique described by Laurell: *Scand. J. Clin. Lab. Invest., 29* (Suppl. 124): 21—37 (1972).

Amidolytic activity of $\alpha_2$M-trypsin complexes in the fluid phase or bound to immobilized rabbit anti-human $\alpha_2$M antibody

The hydrolytic activity of $\alpha_2$M-trypsin complexes were assessed using the chromogenic substrate BzPh-Val-Arg-NHNp. $\beta$-Trypsin was incubated with a mixture of unlabeled and $^{125}$I-labeled $\alpha_2$M at trypsin: $\alpha_2$M molar ratios of 0.6, 0.3, and 0.15, well below the 2 moles of $\beta$-trypsin per mole $\alpha_2$M binding capacity of $\alpha_2$M established in a previous study. The amidolytic activity of these mixtures was found to be linear with time and proportional to the concentration of $\beta$-trypsin in the system. The $\beta$-trypsin-$\alpha_2$M mixtures were also incubated with the IgG fraction of rabbit anti-human $\alpha_2$M antiserum coupled to Bio-Gel A-5m®. After extensive washing the gel pellets were counted in a counter for $^{125}$I and were found to have bound 88 % of the $^{125}$I-$\alpha_2$M radioactivity of the original incubation mixture indicating that the majority of the $^{125}$I-$\alpha_2$M in the incubation mixture has been bound to the immobilized antibody. The solid-phase antibody in the absence of $\alpha_2$M-trypsin mixture did not hydrolyze BzPhe-Val-Arg-NHNp, but the solid-phase $\alpha_2$M antibody which was incubated with trypsin-$\alpha_2$M complexes had gained amidolytic activity that was proportional to the amount of trypsin in the original fluid-phase incubation mixture. Incubation of the solid phase antibody with $\beta$-trypsin in the absence of $\alpha_2$M did not confer amidolytic activity upon the gel nor did the immobilized IgG fraction derived from normal rabbit serum bind either $\alpha_2$M or $\alpha_2$M-trypsin complexes. Thus, the activity gained when immobilized anti-human $\alpha_2$M antibody was incubated with the $\alpha_2$M-trypsin mixtures was a reflection of the specific binding of the $\alpha_2$M-trypsin complexes.

Analysis of the kinetics of the hydrolysis of substrate in the fluid-phase by $\alpha_2$M-trypsin complexes as compared to the solid-phase complexes demonstrated no significant differences (Fig. 1). The Michaelis-Menten constants for the fluid-phase complexes as determined by Lineweaver-Burk plots were Km=0 mM and Vmax −1707 min⁻¹, and of the solid phase α₂M-trypsin complexes, Km=0.078 mM · and Vmax=1700 min⁻¹. This indicates that under the experimental conditions detailed for these assays, the binding of the α₂M-trypsin complex to a surface did not affect its amidolytic activity.

Effect of pH and ionic strength on the amidolytic activity of solid-phase antibody-bound α₂M-trypsin complexes

Both soluble α₂M-trypsin complexes and solid-phase antibody-bound α₂M-trypsin complexes demonstrated a similar peak of amidolytic activity between pH 8.0 and 9.0. At higher pH's, there was a significant decrease in substrate hydrolysis. In other experiments, the effect of variations in sodium chloride concentration (0 to 0.5 M) on the catalytic activity of the immobilized α₂M-trypsin complex was studied. A broad unchanging peak of activity from 0.05 to 0.3 M NaCl was observed with a decrease in amidolytic activity at both 0 and 0.5 M NaCl. Both soluble and immobilized

α₂M-trypsin complexes behaved in a similar manner.

Effect of repetitive assays on the amidolytic activity of solid-phase antibody-bound α₂M-trypsin complexes

In order to assess the stability of the immobilized antibody-bound α₂-trypsin complexes, four portions of the immobilized α₂M antibody were incubated with an α₂M trypsin mixture containing a trace amount of ¹²⁵I-α₂M. After repeated washings, the gels were counted to determine the amount of bound α₂M. The insolublized antibody α₂M-trypsin complex was then assayed for BzPhe-Val-Arg-NHNp hydrolytic activity. This procedure was repeated four times. As illustrated in Table I, the specific activity of the α₂M-trypsin complex, bound to the immobilized antibody, did not change with repeated assays indicating that trypsin itself did not dissociate from the complex in the presence of the chromogenic substrate. There was a small equivalent loss of both α₂M and amidolytic activity with repeated assays reflecting either loss of gel during the washings or elution of the α₂M-trypsin complex.

TABLE I

Effect of repetitive assays on the amidolytic activity of insoluble antibody-bound α₂M-trypsin complexes[a]

| Assay No. | µg α₂M[b] | µmoles/L/min[c] | Specific activity (µmoles/L/min/mgα₂M) |
|---|---|---|---|
| 1 | 6.7±0.17 | 0.79±0.03 | 117.9±5.3 |
| 2 | 6.7±0.16 | 0.76±0.05 | 113.4±5.4 |
| 3 . | 6.5±0.25 | 0.77±0.04 | 118.5±2.4 |
| 4 | 5.1±0.65 | 0.60±0.09 | 117.7±6.3 |

[a] Portions (0.2 ml) of a mixture of α₂M and ¹²⁵I-α₂M (50 µg/ml) and β-trypsin (300 ng) were incubated in quadruplicate for 2 hours with rabbit anti-human α₂M-IgG coupled to Bio-Gel A-5m® (10 µl packed gel). Following washings of each sample four times with PBS, it was subsequently assayed for amidolytic activity by adding Tris-CaCl₂ buffer, pH 8.3 (0.4 ml) and BzPhe-Val-Arg-NHNp (0.7 µm/ml, 0.2 ml) for 15 min at room temperature with mixing by inversion. After each assay, the samples were washed four times in succession and the amidolytic activity redetermined. This assay procedure was repeated a total of four times.

[b] The µg α₂M bound to the beads was calculated as the counts bound/total counts × µgα₂M in the original incubation mixture.

[c] All values in the table represent the mean of four determinations.

Studies on the binding of α₂M in human plasma to immobilized anti-human α₂M antibody

Experiments were designed to test whether the binding of purified α₂M or α₂M-trypsin complexes by solid-phase anti-human α₂M antibody could be extended to a complex biological fluid such as plasma. In the study shown in Fig. 2, the solid-phase α₂M antibody was incubated with serial dilutions of plasma to which ¹²⁵I-α₂M was added. The radioactivity bound to the insoluble antibody was measured. The native α₂M in the starting plasma and the residual α₂M in the plasma supernatant following absorption was also quantitated by electroimmunoassay. The results demonstrates that the binding of the ¹²⁵I-α₂M added to plasma by the immobilized antibody parallels the binding of native plasma α₂M.

Specificity of binding of ¹²⁵I-α₂M added to plasma by the solid-phase α₂M antibody was also tested by incubating plasma containing ¹²⁵I-α₂M with unmodified Bio-Gel A-5m®, or this same gel to which the purified IgG fraction of normal rabbit serum, or the IgG fraction of rabbit antisera against either human albumin or haptoglobin was coupled. Under conditions in which greater than 95 % of the radioactivity became bound to insoluble $\alpha_2M$ antibody, less than 2 % of the $^{125}I$-$\alpha_2M$ radioactivity was associated with these other gel preparations.

The effect of time of incubation of plasma with solid-phase $\alpha_2M$ antibody on the binding of $\alpha_2M$ to the gel was investigated (Fig. 3). The antibody gel was incubated for varying time periods with three different dilutions of plasma which contained 12, 60 and 192 µg $\alpha_2M$ respectively. There was a progressive increase in binding throughout the six hour incubation period at all concentrations of $\alpha_2M$. At the lowest concentration of $\alpha_2M$ (12 µg system), 91.7 % of the $\alpha_2M$ was bound after 6 hours. At the highest concentration of $\alpha_2M$, 49 % or 84 µg of $\alpha_2M$ was bound. This indicates that 1.0 ml of the $\alpha_2M$ antibody gel has the capacity to bind at least 9.4 mg $\alpha_2M$.

Studies on the binding of $\alpha_2M$-trypsin complexes in plasma by solid-phase $\alpha_2M$ antibody

The demonstration that $\alpha_2M$ in plasma could be quantitatively removed by immobilized $\alpha_2M$ antibody made it possible to study the binding of exogenous $^{131}I$-$\beta$-trypsin to $\alpha_2M$ in a plasma system. $^{131}I$-$\beta$-trypsin was added to plasma containing 1.5 mg/ml $\alpha_2M$ to give trypsin concentrations of 0.10 to 1.65 µg/ml plasma. This represents a trypsin/ $\alpha_2M$ molar ratio of 0.002 to 0.035. The plasma also contained a trace amount of $^{125}I$-$\alpha_2M$. Portions of plasma were incubated with solid-phase $\alpha_2M$ antibody for two hours. The pellets were washed, counted for both $^{125}I$ and $^{131}I$ radioactivity and the amidolytic activity measured (Fig. 4). Whereas 86.7 % of the $^{125}I$-$\alpha_2M$ in the incubation mixture was bound to the antibody containing gel, only 50.3 % of $^{131}I$-$\beta$-trypsin was similarly bound suggesting that trypsin also binds to other plasma proteins. The percent binding of both $\alpha_2M$ and trypsin was unaffected by the varying concentrations of trypsin added to the plasma. The $^{131}I$ radioactivity bound to the solid-phase antibody was proportional to the amount of $^{131}I$-trypsin in the original mixture.

To control for endogenous amidolytic activity, the solid-phase $\alpha_2M$ antibody was incubated with a plasma $^{125}I$-$\alpha_2M$ mixture to which no $^{131}I$-trypsin had been added. The resulting $\alpha_2M$ antibody gel hydrolyzed 2.4 µmoles substrate/L/min/mg $\alpha_2M$ corresponding to the addition to plasma of approximately 142 ng $\beta$-trypsin/ml. This background activity was subtracted from the amidolytic activity of the gels incubated with the plasma, trypsin mixtures. The amidolytic activity of the $\alpha_2M$ antibody gels incubated with the $^{131}I$-trypsin, plasma mixtures was proportional to the initial concentration of trypsin added, and parallel to the $^{131}I$ counts bound to the gel (Fig. 4).

Analysis of a $^{131}I$-$\beta$-trypsin, plasma mixture by molecular sieve chromatography

From a comparison of the binding of $\alpha_2M$ to the solid-phase $\alpha_2M$ antibody in the experiment illustrated in Fig. 4 (86.7 %), and the binding of $^{131}I$-trypsin (50.3 %), it can be calculated that the total $\alpha_2M$ in plasma should have bound 58.0 % of the trypsin added to the plasma. Thus it seemed likely that approximately 42 % of the trypsin may have been complexed to other plasma proteins and therefore unavailable to the $\alpha_2M$. To verify this possibility, a mixture of $^{131}I$-trypsin, $^{125}I$-$\alpha_2M$ and plasma was fractionated by molecular sieve chromatography (Fig. 5). The major $^{131}I$-trypsin peak, containing 57.4 % of the total $^{131}I$ counts, co-eluted with the $^{125}I$-$\alpha_2M$ in the first, high molecular weight protein peak. The second $^{131}I$-trypsin peak contained 40.3 % of the total $^{131}I$ counts and eluted earlier than the albumin peak at a position consistent with complex formation between trypsin and a plasma protein.

To further confirm the specificity of the insoluble $\alpha_2M$ antibody, $\alpha_2M$-trypsin interaction and to identify the nature of the $^{131}I$-trypsin peaks from the gel filtration column, 0.6 ml of each peak fraction of $^{131}I$-trypsin was incubated with the solid phase $\alpha_2M$ antibody (0.1 ml packed gel). Following an 18 h incubation, 99 % of the $^{125}I$-$\alpha_2M$ and 94 % of the $^{131}I$-trypsin of the $\alpha_2M$ peak fraction 36 was bound to the immobilized antibody. Less than 5 % of $^{131}I$-trypsin was bound to the antibody gel in the incubation mixture of the second $^{131}I$-trypsin peak (fraction 46). Therefore most of the $^{131}I$-trypsin in the first high molecular weight peak was complexed with $\alpha_2M$. The failure of $^{131}I$-trypsin in the second radioactive peak to bind to the $\alpha_2M$ antibody gel provides further evidence that trypsin had reacted with a protein other than $\alpha_2M$.

The process of the invention make possible the study of enzymes and inhibitor-enzyme complexes present in biological fluid, broadly and specifically presents a new approach to the study of the physiologic function of human plasma $\alpha_2$-macroglobulin. The method of the invention makes it possible to further characterize inhibitor-enzyme complexes and particularly the protease binding capacity of $\alpha_2M$ in both purified systems and in complex biological fluids. As exemplified the solid-phase antibody-bound $\alpha_2M$-$\beta$-trypsin complexes retain their ability to hydrolyze the tripeptide chromogenic substrate BzPhe-Val-Arg-NHNp with Michaelis-Menten constants identical to those of the soluble complexes. This indicates that the binding of the $\alpha_2M$-trypsin complex to an immobilized antibody does not appreciably effect the amidolytic activity of the bound protease. The antibody-bound $\alpha_2M$-trypsin complexes proved to be remarkably stable since they remain associated with the agarose gel matrix through repeated washings and assays of amidolytic activity. The finding that trypsin remains bound to $\alpha_2M$ in the presence of substrate provides further evidence that trypsin binds to $\alpha_2M$ at a site different from its catalytic site.

Studies of the conditions required to optimize substrate hydrolysis as in the Example showed that the optimum pH of the reaction for both the

soluble $\alpha_2$M-trypsin complex as well as the solid-phase, antibody-bound complex was between 8 and 9. In contrast, Rindernecht, et al., *Biochem. Biophys. Acta, 377:* 158—165 (1975), using a different chromogenic substrate, N-carbobenzoxy-glycyl-glycyl-L-arginine-2-naphthyl-amide HCl, found that catalysis was greatest at pH 10.0. They postulated that this degree of alkalinity was necessary to alter the microvironment of the trypsin active site within the $\alpha_2$M-trypsin complex so that the substrate could be hydrolyzed at a rate similar to that of free trypsin. It is not clear whether the difference in pH optimum between their study and our reflects the behaviour of the purified $\beta$-trypsin used in the present study, the difference in substrate, or other factors.

We have demonstrated that the binding of $\alpha_2$M-trypsin complexes by immobilized $\alpha_1$M antibody also occurs when the assay system is applied to human plasma. Prior studies by Haverback et al., *J. Clin. Invest.,* 41: 972—980 (1962), was formed between trypsin and a serum protein that possessed an $\alpha_2$ electrophoretic mobility. Mehl et al., *Science, 145:* 821—822 (1964) provided direct evidence that $\alpha_2$M was the protein responsible for the enzyme binding activity. In the present study, when plasma containing a trace quantity of $^{125}$I-$\alpha_2$M was incubated with immobilized $\alpha_2$M antibody, both radiolabeled and native $\alpha_2$M were bound. The electroimmunoassay technique of Laurell was used to establish that the binding of $^{125}$I-$\alpha_2$M to the solid-phase $\alpha_2$M antibody was proportional to the amount of unlabeled native $\alpha_2$M bound. The addition of the radiolabeled antigen to plasma made it possible, therefore, to quantitate the binding of plasma $\alpha_2$M to the solid-phase antibody. The binding of plasma $\alpha_2$M to the insoluble gel containing the antibody was specific since other immobilized antibodies such as anti-albumin, antihaptoglobin, or the IgG fraction of normal rabbit serum did not bind significant amounts of plasma $\alpha_2$M.

To establish that the solid-phase antibody technique detailed in this study could detect $\alpha_2$M-enzyme complexes in a biological fluid such as plasma, varying concentrations of $^{131}$I-$\beta$-trypsin were added to plasma that contained a trace quantity of $^{125}$I-$\alpha_2$M. Subsequent assay demonstrated that $\beta$-trypsin was found to the immobilized $\alpha_2$M antibody and that the amount of $^{131}$I-trypsin bound and the amidolytic activity associated with the solid-phase antibody was linearly related to the concentration of trypsin originally added to the plasma. Since control studies established that $^{131}$I-$\beta$-trypsin itself did not bind the antibody gel, the trypsin activity that we observed was a specific measure of $\alpha_2$M-trypsin complexes. At each concentration of trypsin added to plasma, 58 % was bound to $\alpha_2$M, a finding explained by gel filtration studies that demonstrated that the remainder of the trypsin was associated with another protein, possibly $\alpha_1$-antitrypsin. These results parallel the findings of Ganrot, *Arkiv for Kemi Bd 26*, No. 50: 577—582 (1967), who demonstrated that the affinity of trypsin for $\alpha_2$M was greater than that for other trypsin inhibitors in plasma.

$\alpha_2$M is a unique plasma protease inhibitor in that it forms a complex with an exceptionally wide variety of proteases including serine, thiol, carboxyl and metalloproteases derived from plasma, cells, or from microorganism; [Harpel et al. (1976, *supra*, Starkey et al., supra]. Complexes between $\alpha_2$M and several different proteases have been identified in plasma; [Harpel et al., *J. Clin. Invest.* 52: 2175—2184 (1973); Nilehn et al., *Scand. J. Clin. Lab. Invest.*, 20: 113—121 (1967)], in peritoneal, [Ohlsson, *Bayer Symp. V., Proteinase Inhibitors;* Fritz et al., eds. Springer Verlag, Berlin (1974) pp. 96—105], pleural [Bieth et al., *Clin. Chim. Acta, 22:* 639—642 (1968); Bieth et al., *Clin. Chim. Acta, 30;* 621—626 (1970)], and in synovial effusions [Shtacher et al., *Biochim. Biophys. Acta, 303:* 138—147 (1973); Abe et al., J. Biochem (Tokyo), *71:* 919—922 (1973); Abe et al., *J. Biochem.* (Tokyo), 73: 897—900 (1973)], however a convenient method suitable for the quantitative analysis of these complexes has not previously been available. The solid-phase $\alpha_2$M antibody technique described in this study facilitates the rapid isolation and concentration of enzyme-enzyme inhibitor complexes generally of $\alpha_2$M-protease complexes specifically and the measurement of their presence and/or catalytic capacity in biological fluids in various physiologic and pathologic states. Modifications of the assay conditions will permit the measurement of the activity of physiologically important enzymes and enzyme inhibitors, particularly proteases such as thrombin, plasmin and plasma kallikrein when bound to $\alpha_2$M.

**Claims**

1. A method for analyzing biological fluids for proteolytic enzyme inhibitor-proteolytic enzyme complexes which consists of:
   a) forming an immobilized immunocaptured enzyme inhibitor enzyme complex comprising enzyme, enzyme inhibitor and enzyme inhibitor antibody or a reactive fragment thereof, and
   b) detecting the enzyme or enzyme inhibitor-enzyme complex in the immobilized immunocaptured complex whereby the analytical determination comprises reacting the immobilized complex against substrates reactive to the captured enzyme when the captured enzyme inhibitor enzyme complex is enzymatically reactive or whereby the analytical determination comprises reacting the immobilized complex with a detection facilitating material which reacts with a site specific to the bound enzyme or the enzyme inhibitor enzyme complex when the captured enzyme inhibitor-enzyme complex is not enzymatically reactive.

2. The method of claim 1 wherein immobilized enzyme inhibitor, enzyme complex is affixed to a matrix by means of the enzyme inhibitor antibody or active fragment thereof.

3. The method of claims 1 or 2 wherein the enzyme inhibitor is $\alpha_2$-macroglobulin.

4. The method of claims 1 or 2 wherein the enzyme inhibitor is $\alpha_2$-macroglobulin and the matrix is an agarose gel.

5. A method which consists of:

a) affixing a proteolytic enzyme inhibitor antibody or reactive fragment thereof to a matrix

b) contacting the resultant inhibitor reactive matrix with a biological fluid to capture enzyme inhibitor-enzyme complex from said fluid and

c) analytically determining the identity or quantity of the captured enzyme, the analytical determination comprises reacting the immobilized complex against substrates reactive to the captured enzyme when the captured enzyme inhibitor-enzyme complex is enzymatically reactive and the analytical determination comprises reacting the immobilized complex with a detection facilitating material which reacts with a site specific to the bound enzyme or the enzyme inhibitor-enzyme complex when the captured enzyme inhibitor-enzyme complex is not enzymatically reactive.

6. The method as in claim 5 wherein the enzyme inhibitor is $\alpha_2$-macroglobulin.

7. The method of claims 1 and 2 wherein the biological fluid is a human biological fluid.

8. The method of claim 5, wherein the biological fluid is a human biological fluid.

**Patentansprüche**

1. Verfahren zu Analyse biologischer Flüssigkeiten für proteolytische Enzym-Inhibitorproteolytische Enzym-Komplexe, dadurch gekennzeichnet, daß man

(a) einen immobilisierten, immunogefangenen Enzym-Inhibitor-Enzym-Komplex bildet, der Enzym, Enzym-Inhibitor und Enzym-Inhibitor-Antikörper oder ein reaktives Fragment davon enthält, und

(b) das Enzym oder den Enzym-Inhibitor-Enzym-Komplex in dem immobilisierten, immunogefangenen Komplex nachweist, wobei die analytische Bestimmung die Umsetzung des immobilisierten Komplexes gegen Substrate, die gegenüber dem gefangenen Enzym reaktiv sind, wenn der gefangene Enzym-Inhibitor-Enzym-Komplex enzymatisch reaktiv ist, umfaßt oder bei dem die analytische Bestimmung die Umsetzung des immobilisierten Komplexes mit einem Material, das den Nachweis erleichtert, umfaßt, und wobei das Material mit einer Stelle reagiert, die spezifisch für das gebundene Enzym oder den Enzym-Inhibitor-Enzym-Komplex ist, wenn der gefangene Enzym-Inhibitor-Enzym-Komplex enzymatisch nicht reaktiv ist.

2. Verfahren nach Anspruch 1, dadurch gekennzeichnet, daß der immobilisierte Enzym-Inhibitor-Enzym-Komplex an eine Matrix mittels des Enzym-Inhibitor-Antikörpers oder eines aktiven Fragments davon gebunden ist.

3. Verfahren nach einem der Ansprüche 1 oder 2, dadurch gekennzeichnet, daß der Enzym-Inhibitor $\alpha_2$-Makroglobulin ist.

4. Verfahren nach einem der Ansprüche 1 oder 2, dadurch gekennzeichnet, daß der Enzym-Inhibitor $\alpha_2$-Makroglobulin ist und daß die Matrix ein Agarosegel ist.

5. Verfahren, dadurch gekennzeichnet, daß mas

(a) einen proteolytischen Enzym-Inhibitor-Antikörper oder ein reaktives Fragment davon an eine Matrix bindet,

(b) die entstehende, reaktive Inhibitor-Matrix mit einer biologischen Flüssigkeit behandelt, um den Enzym-Inhibitor-Enzym-Komplex aus der Flüssigkeit abzufangen, und

(c) die Identität oder Menge des abgefangenen Enzyms analytisch bestimmt, wobei die analytische Bestimmung die Reaktion des immobilisierten Komplexes gegenüber Substraten, die gegenüber dem abgefangenen Enzym reaktiv sind, umfaßt, wenn der abgefangene Enzym-Inhibitor-Enzym-Komplex enzymatisch reaktiv ist, und wobei die analytische Bestimmung die Umsetzung des immobilisierten Komplexes mit einem den Nachweis erleichternden Material umfaßt, welches mit einer Stelle reagiert, die spezifisch für das gebundene Enzym oder den Enzym-Inhibitor-Enzym-Komplex ist, wenn der abgefangene Enzym-Inhibitor-Enzym-Komplex enzymatisch nicht reaktiv ist.

6. Verfahren nach Anspruch 5, dadurch gekennzeichnet, daß der Enzym-Inhibitor $\alpha_2$-Makroglobulin ist.

7. Verfahren nach Anspruch 1 oder 2, dadurch gekennzeichnet, daß die biologische Flüssigkeit eine humane, biologische Flüssigkeit ist.

8. Verfahren nach Anspruch 5, dadurch gekennzeichnet, daß die biologische Flüssigkeit eine humane, biologische Flüssigkeit ist.

**Revendications**

1. Procédé d'analyse de fluides biologiques pour la détermination de complexes d'enzyme protéolytique-inhibiteur d'enzyme protéolytique, caractérisé en ce qu'il comprend:

a) la formation d'un complexe d'enzym-inhibiteur d'enzyme immunocapté immobilisé, comprenant une enzyme, un inhibiteur d'enzyme et un anticorps de l'inhibiteur d'enzyme ou un fragment réactif de celui-ci, et

b) la détection de l'enzyme ou du complexe d'enzyme inhibiteur d'enzyme dans le complexe immunocapté et immobilisé selon laquelle la détermination analytique comprend la réaction du complexe immobilisé avec des substances réactives vis à vis de l'enzyme captée lorsque la complexe d'enzyme-inhibiteur d'enzyme capté est enzymatiquement réactif ou, la détermination analytique comprend la réaction du complexe immobilisé avec une matière facilitant la détection, qui réagit avec un site spécifique de l'enzyme fixée ou du complexe d'enzyme-inhibiteur d'enzyme lorsque la complexe d'enzyme-inhibiteur d'enzyme capté n'est pas enzymatiquement réactif.

2. Procédé suivant la revendication 1, caractérisé en ce que le complexe d'enzyme-inhibiteur d'enzyme est fixé à une matrice au moyen de l'anticorps de l'inhibiteur d'enzyme ou d'un fragment actif de celui-ci.

3. Procédé suivant la revendication 1 ou 2, caractérisé en ce que l'inhibiteur d'enzyme est de l'$\alpha_2$-macroglobuline.

4. Procédé suivant la revendication 1 ou 2, caractérisé en ce que l'inhibiteur d'enzyme est de l'$\alpha_2$-macroglobuline et la matrice est un gel d'agarose.

5. Procédé caractérisé en ce qu'il comprend:

a) la fixation d'un anticorps de l'inhibiteur d'enzyme protéolytique ou d'un fragment réactif de celui-ci à une matrice.

b) La mise en contact de la matrice résultante réactive vis à vis de l'inhibiteur avec un fluide biologique pour capter le complexe d'enzyme-inhibiteur d'enzyme présent dans le fluide, et

c) la détermination analytique de l'identité ou de la quantité de l'enzyme captée, la détermination analytique comprenant la réaction du complexe immobilisé avec des substrats réactifs vis-à-vis de l'enzyme captée lorsque le complexe d'enzyme-inhibiteur d'enzyme capté est réactif du point de vue enzymatique et la détermination analytique comprenant la réaction du complexe immobilisé avec une matière facilitant la détection qui réagit avec un site spécifique de l'enzyme fixée ou du complexe d'enzyme-inhibiteur d'enzyme, lorsque le complexe d'enzyme-inhibiteur d'enzyme capté n'est pas enzymatiquement réactif.

6. Procédé suivant la revendication 5, caractérisé en ce que l'inhibiteur d'enzyme est l'$\alpha_2$-macroglobuline.

7. Procédé suivant la revendication 1 ou 2, caractérisé en ce que le fluide biologique est un fluide biologique humain.

8. Procédé suivant la revendication 5, caractérisé en ce que le fluide biologique est un fluide biologique humain.

FIG. 1

FIG 2

FIG. 3

FIG. 4

2

FIG. 5